# EUROPEAN PATENT SPECIFICATION

(11) **EP 2 658 434 B1**
(45) Date of publication and mention of the grant of the patent: **02.12.2015**
(21) Application number: 10798150.8
(22) Date of filing: 30.12.2010
(51) Int. Cl.: G01P 15/08, A61B 5/11, A61B 5/00, A63B 24/00

(54) **METHOD FOR CONFIGURING A MOTION SENSOR AS WELL AS A CONFIGURABLE MOTION SENSOR AND A SYSTEM FOR CONFIGURING SUCH A MOTION SENSOR**
VERFAHREN ZUM KONFIGURIEREN EINES BEWEGUNGSSENSOR SOWIE KONFIGURIERBARER BEWEGUNGSSENSOR UND SYSTEM ZUR KONFIGURATION EINES DERARTIGEN BEWEGUNGSSENSORS
PROCÉDÉ DE CONFIGURATION D'UN CAPTEUR DE MOUVEMENT, CAPTEUR DE MOUVEMENT CONFIGURABLE ET SYSTÈME DE CONFIGURATION D'UN TEL CAPTEUR DE MOUVEMENT

(43) Date of publication of application: 06.11.2013
(73) Proprietor: AR Innovation AG, 8712 Stäfa (CH)
(72) Inventor: BAECHLER, Herbert, CH-8008 Zürich (CH)
(74) Representative: Werner, André
(86) International application number: PCT/EP2010/070914
(87) International publication number: WO 2012/052070

(56) References cited:
- EP-A1- 2 179 770
- WO-A1-2008/058959
- WO-A1-2008/132105
- US-A1- 2003 004 424
- US-A1- 2007 197 878
- US-A1- 2009 319 221
- US-B1- 7 602 301

## Description

### TECHNICAL FIELD

The present invention relates to motion sensors, especially to a versatile motion sensor that can be used for a wide variety of sports, fitness, ambulatory monitoring and therapy applications. The present invention specifically pertains to a method for configuring such a versatile motion sensor, a corresponding configurable motion sensor as well as a system for configuring such a motion sensor.

### BACKGROUND OF THE INVENTION

Motion sensors have become very popular in sports for determining various performance measures. In cycling small computers on the handle bar are very common for providing information to a rider about elapsed time, distance travelled, speed and pedalling cadence. In jogging elapsed time, distance travelled, speed and pace are mostly of interest. Very different physical methods are employed to measure and determine the data of interest.

Accelerometers are applied to bicycles for various purposes, e.g. in inclinometers, speed and cadence sensors, and for determining crank and pedal position, pedalling power, etc. Meanwhile, miniature accelerometers suitable for these tasks are cheap mass products that are readily available from manufacturers such as Analog Devices, Inc. or VTI Technologies. Furthermore, miniature gyroscopes, which measure angular velocity, can also be used for motion sensing. Such devices are available for instance from Murata Manufacturing Co., Ltd.

US 4,526,036 discloses a cadence meter comprising means for measuring acceleration intended to be mounted on a nonrotating part of a bicycle. The cadence is determined based on measuring the alternating phases of acceleration and de-acceleration of the bicycle in its direction of travel caused by the changing force applied to the pedals by the bicyclist during each pedalling cycle. A similar technique is employed in the measurement device described in EP 1 213 561 B1. This measurement device includes an accelerometer, which is mounted on a bicycle such that its measurement axis coincides with the direction of travel. The output signal of the accelerometer is processed in order to extract the frequency of pedalling, i.e. the pedalling cadence. Furthermore, WO 2008/058164 A2 presents a crank set based bicycle power measuring device wherein the pedalling cadence is required as part of the power calculation. In conjunction with determining the pedalling cadence, it is mentioned that an accelerometer mounted on a crank set can be used to measure the direction of gravity relative to the orientation of the crank. In WO 2010/000369 A1 the use of an accelerometer in a device for measurement of cycling power output is described, wherein the accelerometer is embedded in a bicycle cleat bolted to a bicyclist shoe or alternatively mounted on a pedal of a bicycle or a leg or foot of a bicyclist. It is suggested to determine the cadence based on the elapsed time between measuring consecutive minimum and maximum values, respectively, of the output signals from the accelerometer, which occur at the top dead centre and bottom dead centre, respectively, of each revolution of the cranks. Mavic, a manufacturer of bike systems and rider's equipment, employs accelerometers in its "Smart Cadence" pedalling cadence sensor which are integrated into a stretch fabric that is worn at the bicyclist's ankle. In the international application PCT/EP2010/065537 the present inventor proposes a method for determining both a bicyclist's pedalling cadence as well as a bicycle's travelling speed from the outputs of a 2/3-axis accelerometer arranged at a wheel of a bicycle.

Jogging sensors, like the stride sensors provided by Polar or Nike, employ accelerometers to determine distance and speed from the pace of steps. Such a solution is described in US 6,018,705. The time period a foot is in contact with the ground during a stride taken by a jogger and the period that the foot is not in contact with the ground between strides taken by the jogger are determined by processing and analysing the output signals of an accelerometer.

Accelerometer-based motion sensors can also be applied in a wide variety of other sports such as rowing, skiing, cross-country skiing, golfing, exercising, swimming, ice skating, rollerblading, etc., to analyse characteristic movements and activities of an athlete. The computation of associated performance measures is often less straight forward than counting steps or detecting wheel rotations, but is well-known by those skilled in the art as evidenced by a large body of patent literature related to this topic. A few representative examples from different sports of motion sensors which employ accelerometers are given in the following. US 7,689,378 B2 describes a motion sensing apparatus utilising a tri-axial accelerometer together with a tri-axial gyroscope as well as a tri-axial magnetometer and discloses techniques for analysing a golf stroke. US 2010/0204952 A1 describes a portable wrist worn device for determining information about the movement of a human body when swimming. WO 98/42413 A1 discloses an exercise monitoring system. WO 01/10508 A1 describes a rehabilitation device. US 6,980,118 B2 discloses a method and apparatus for measuring stroke rating in rowing.

Furthermore, US 2009/0319221 A1 discloses an electronic device that monitors accelerations using an inertial sensor. The electronic device identifies a current motion state based on the accelerations. Additionally, the electronic device determines an application that subscribes to a motion state identification service and notifies the application of the current motion state. Moreover, US 7,602,301 B1 discloses apparatus, systems, and methods for measuring and analysing movements of a body and for communicating information related to such body movements over a network. In certain embodiments, a system gathers biometric and biomechanical data relating to positions, orientations, and movements of various body parts of a user performed during sports activities, physical rehabilitation, or military or law enforcement activities. The biometric and biomechanical data can be communicated to a local and/or remote interface, which uses digital performance assessment tools to provide a performance evaluation to the user. The performance evaluation may include a graphical representation (e.g., a video), statistical information, and/or a comparison to another user and/or instructor. In some embodiments, the biometric and biomechanical data is communicated wirelessly to one or more devices including a processor, display, and/or data storage medium for further analysis, archiving, and data mining. In some embodiments, the device includes a cellular telephone.

In the numerous applications indicated above the motion sensing device is basically always the same, namely comprising a sensor element in the form of an accelerometer and/or a gyroscope to measure motion, a processing element to process the output of the sensor element and to determine a performance measure therefrom, a communication element to send the determined data/information from the location of the sensor to a remote unit where it is displayed to a user. The difference between individual motion sensor devices essentially resides in the processing algorithms used to compute the desired performance measure and the attachment means which enable secure fixation of the motion sensor device at an appropriate measurement location, i.e. on a part of the body of an athlete or on a part of a piece of sports equipment being used by an athlete.

As a consequence of the fact that each application requires specific attachment means for attaching the motion sensor device to the measurement location and a dedicated processing algorithm to determine a specific performance measure, an athlete, such as a triathlete, carrying out a variety of sports will require a multitude of different motion sensor devices to cover all practised sport disciplines, e.g. swimming, cycling and running.

### SUMMARY OF THE INVENTION

It is an object of the present invention to provide a versatile motion sensor which can be used for a wide variety of sports, fitness, ambulatory monitoring and therapy applications.

At least this object is achieved by a motion sensor which is configurable according to the method of claim 1. Preferred embodiments of the proposed method, a corresponding configurable motion sensor, a sensor network as well as a system for configuring such a motion sensor are given in the further claims.

A method is provided for configuring a motion sensor comprising an accelerometer and/or a gyroscope, a processing unit, a memory unit, a program selection means, a wireless transmitting unit, and a receiving unit, the method comprising:
- providing configuration data to the program selection means;
- selecting by the program selection means a signal processing program from a plurality of signal professing programs stored in the memory unit dependent on the provided configuration data; and
- determining by the processing unit at least one motion parameter from one or more output signals of the accelerometer and/or the gyroscope using the selected signal processing program.

In this way the motion sensor is adapted to a specific application by providing configuration data to it. The configuration data determines the way in which the one or more output signals of then accelerometer and/or the gyroscope are processed in order to obtain at least one motion parameter. To achieve this an appropriate signal processing program for processing the one or more output signals of the accelerometer and/or the gyroscope to obtain at least one desired motion parameter for a specific application is selected from a plurality of signal processing programs stored in the memory unit of the motion sensor based upon the configuration data. By doing so the signal processing applied to determine the desired motion parameter(s) is adapted to the application at hand, e.g. dependent on the arrangement of the motion sensor at the athlete or sports equipment.

Possible examples of motion parameters that are determined by the motion sensor depending on the application at hand are stride speed, acceleration, velocity, stride distance, total distance, gait/pedalling efficiency, power, energy, (maximum) impact, (average) calories consumed, maximum speed, speed variability, a cadence associated with pedalling, rowing, walking, cunning, cross-country skiing, roller skating, inline skating, ice skating, arm swinging or swimming stroke, pinching speed and impact, springiness, striking, drive speed or throwing speed.

The method further comprises:
- selecting a transmission protocol program from a plurality of transmission protocol programs stored in the memory unit dependent on the provided configuration data; and
- sending by the wireless transmitting unit the at least one motion parameter or the one or more output signals of the accelerometer and/or the gyroscope or one or more signals based on said output signals using the selected transmission protocol program.

In this way the configuration data determines the way in which the at least one motion parameter is sent to a remote device such as a sports computer, e.g. for displaying the at least one motion parameter to the user or for -its further processing or storage. An appropriate transmission protocol program for sending the at least one motion parameter to the remote device is selected from a plurality of transmission protocol programs stored in the memory unit of the motion sensor based upon the configuration data. By doing so the transmission is adapted to the- kind of information being determined by the motion sensor, e.g. the data format in and the data rate at which the desired motion parameter(s) is/are being produced by the motion sensor, and/or to the kind of transmission link being employed, e.g. in terms of range, speed, power, transmission channel characteristics., etc.

The method further comprises receiving by the deceiving unit a signal processing program and/or a transmission protocol program.

In this way a further signal processing program, which is not yet stored in the memory unit of the motion sensor, is downloaded to the motion sensor. By doing so the motion sensor is able to determine one or more motion parameters for a new application that it was not able to handle before. Likewise, a further transmission protocol program, which is not yet stored in the memory unit of the motion sensor, is downloaded to the motion sensor. By doing so the motion sensor is able to support a new transmission protocol that it was not able to execute before.

In a further embodiment of the method, the step of providing configuration data comprises:
- receiving by the receiving unit the configuration data.

In this way the configuration data is provided to the motion sensor from a separate device via the receiving unit. By doing so the motion sensor is configured according to configuration data input to or determined at a separate, e.g. remote, device for instance as desired by user of the motion sensor.

In yet a further embodiment of the method, wherein the motion sensor further comprises a motion classification means, the method further comprises:
- extracting motion features from the one or more output signals of the accelerometer and/or the gyroscope;
- identifying by the motion classification means a motion pattern from a plurality of motion patterns based on the extracted motion features; and
- generating at least part of the configuration data to be provided to the program selection means dependent on the identified motion pattern.

In this way the configuration data is generated by the motion sensor itself based on a motion pattern that the motion classification means has identified from a plurality of motion patterns. By doing so the motion sensor is automartically configured, i.e. it configures ïtself, according to the application for which it is being used by identifying the type of motion the motion sensor is sensing. Thus, the user does not need to provide this configuration data to the motion sensor. In this way the motion sensor can also automatically adapt itself to new applications based on motions patterns which are similar to known motion patterns of applications to which the motion sensor has been applied before;

Motion pattern classification is well documented for instance in "M. J. Mathie, B. G. caller, N. H. Lovell and A. C. F. Coster, classification of basic daily movements using a triaxial accelerometer, Med. Biol. Eng. Comput., 2004, 42, pp. 679 - 687" and "A. Mannini and A. M. Sabatini, Machine learning methods for classifying human physical activity from on-body accelerometers, Sensors, 2010, 10, pp. 1154-1175" as well as the publications cited therein.

In yet a further embodiment the method further comprises:
- downloading a software module, which comprises at least a signal processing program and a transmission protocol program, from an application server to a sensor interfacing device or sensor hub, such as for instance a sports computer, a mobile phone or a portable digital assistant;
- extracting the signal processing program and/or the transmission protocol program from the software module; and
- sending the signal processing program and/or the transmission protocol program from the sensor interfacing device or sensor hub to the receiving unit.

In this way the signal professing program and/or the transmission protocol program is provided to the motion sensor from an application server via an intermediary sensor interfacing device or sensor hub, which is operationally connected to the receiving unit of the motion sensor. Different software modules, for different applications can be provided by the application server. The user is able to select the desired application and the associated software module is then downloaded from the application server by the sensor interfacing device or sensor hub, which extracts the signal processing program and/or the transmission protocole program from the software module and sends either or both of them to the receiving unit of the motion sensor.

In yet a further embodiment the method further comprises receiving by the sensor hub using the transmission protocol program the at least one motion parameter or the one or more output signals of the accelerometer and/or the gyroscope or one or more signals based on said output signals from the wireless transmitting unit.

In this way the sensor hub receives data from the wireless transmitting unit by employing the same transmission protocol for receiving the data as the wireless transmitting unit is using to send the data. This is for instance achieved by extracting the transmission protocole program from the software module downloaded from the application server and distributing the transmission protocol program to both the receiving unit of the motion sensor as well as the sensor hub. By doing so it is always ensured that dependent of the kind of data and kind of transmission required by the application the appropriate transmission protocol program is being used by the wireless transmission unit of the motion sensor and the sensor hub.

In yet a further embodiment of the method, wherein the software module further comprises an application program, the method further comprises extracting the application program from the software module and executing by the sensor hub the application program for post-processing and/or displaying and/or storing the received at least one motion parameter or the one or more output signals of the accelerometer and/or the gyroscope or one or more signals based on said output signals from the wireless transmitting unit.

In this way the sensor hub is able to suitably post-process and/or display and/or store dependent on the application at hand the data sent by the wireless transmitting unit. Furthermore by distributing the application program used by the sensor hub to perform the latter as part of the sortware module containing the signal processing program and the transmission protocol program it is ensured that the correct program is available at the sensor hub to receive and post-process/display/store the data sent by the wireless transmitting unit.

In yet a further embodiment the method further comprises sending by the wireless transmitting unit information regarding one or more of the following to the sensor hub:
- configuration data;
- identification number, such as a serial number or address data, of the motion sensor;
- list of signal processing programs stored in the memory unit;
- list of transmission protocol programs stored in the memory unit;
- information regarding one or more of the signal processing programs or one or more of the transmission protocol programs stored in the memory unit, for instance a checksum or a version number;
- information regarding the selected signal processing program or the selected transmission protocol program.

In this way the sensor hub is provided with knowledge of the configuration data which is presently being utilised by the motion sensor, so that new on configuration data can be sent by the sensor hub if the application is to be altered. Furthermore, -it is possible to determine the identity of the motion sensor by being provided with e.g. its serial number or some sort of address data. Knowledge of the signal processing programs and/or the transmission protocol programs stored in the memory unit is important in order to determine if an additional signal processing program and/or an additional transmission protocole program needs to be uploaded to the memory unit when for instance the motion sensor is to be applied in a new application. Moreover, information such as a checksum can be employed to determine if a certain program was uploaded correctly or corrupted due to transmission errors. Furthermore, information such as a program version number can be used to determine if a program stored in the memory unit is still up-to-date or uploading of the most recent version is necessary.

Moreover, information regarding the presently selected signal processing program or the presently selected transmission protocol program can for instance be used to check if the correct program (s) was/were selected dependent on the configuration data which was provided. This information is for instance also relevant when the motion sensor has configured itself after having identified a specific motion pattern. In this way the sensor hub can adapt its transmission protocol program and/or application program to the data being provided by the wireless transmitting unit.

As a further aspect and in correspondence to the proposed method, a configurable motion sensor is provided comprising an accelerometer and/or a gyroscope, a processing unit, a memory unit, a wireless transmitting unit, a receiving unit and a program selection means, wherein the processing unit is connected to the accelerometer and/or the gyroscope, the memory unit, the wireless transmitting unit, the receiving unit and the program selection means. In such a configurable motion sensor the receiving unit is operable to receive configuration data and the processing unit is operable to determine at least one motion parameter from one or more output signals of the accelerometer and/or the gyroscope using one of a plurality of signal processing programs storable in the- memory unit .and selectable by the program selection means dependent on the configuration data. Moreover, the wireless transmitting unit is operable to transmit the at least one motion parameter using one of a plurality of transmission protocol programs storable in the memory unit and selectable by the program selection means dependent on the configuration data. Moreover, the receiving unit is operable to received at least one of the plurality of signal processing programs and/or at least one of the plurality or transmission protocol programs.

In a further embodiment the configurable motion sensor further comprises a separate sensor hub, such as for instance a sports computer, a mobile phone or a personal digital assistant, wherein the wireless transmitting unit is wirelessly connected to the sensor hub, and wherein the sensor hub is adapted to perform one or more of the following:
- present the at least one motion parameter to the user of the sensor hub, for instance visually via a display or acoustically, e.g. via a loudspeaker;
- send the at least one motion parameter to a remote computer or a data server via a communication network, e.g. by means of short message service or packet data service;
- present a time series of the least one motion parameter to the user of the sensor hub via a display;
- store the at least one motion parameter in a memory of the sensor hub.

In this way the data sent by the wireless transmitting unit is made available to the user in a number of different ways. For instance in numeric format to display the momentary value of a motion parameter, e.g. speed, which is continuously updated as new data is received. The received data can also be sent to a remote computer or data server for storage and later evaluation, or e.g. for monitoring by a coach/trainer, who may for instance be analyzing performance data from a number of different athletes simultaneously during a training session. Furthermore, data in a certain time window, e.g. present as well as some preceding data, can be displayed at the sensor hub, or data from a previous training session can be constantly compared with the data from the present training session. In order to do the latter the received data is continuously stored in the sensor hub for later use.

In yet a further embodiment of the configurable motion sensor the sensor hub is connected to the receiving unit via a wireless connection or via a wired connection and the sensor hub is further adapted to perform one or more of the following:
- send configuration data;
- send a signal processing program;
- send a transmission protocol program;
to the receiving unit.

In this way configuration data and/or a signal processing program and/or a transmission protocol program can be provided to the receiving unit by connecting the sensor hub to the receiving unit directly by means of a cable thus providing a fast link appropriate for transferring large programs to the memory unit. Alternatively, small amounts of configuration data can be provided to the receiving unit via a wireless link from the sensor hub allowing very convenient and rapid reconfiguration of the motion sensor when the user wants to switch to a different application., e.g. a triathlete switching from cycling, where pedalling cadence was being measured by the motion sensor, to running, where the running pace is to be determined by same motion sensor perhaps being relocated from a part of the bicycle to a part of the triathlete's body.

In yet a further embodiment the configurable motion sensor further comprises motion classification means capable of identifying a plurality of motion patterns based on motion features extractable from the one or more output signals of the accelerometer and/or the gyroscope.

In yet a further embodiment of the configurable motion sensor the motion classification means is connected to the program selection means and determines the signal processing program and/or the transmission protocol program to be employed for determining the at least one motion parameter and/or for transmitting the at least one motion parameter, respectively.

In this way "self-configuration" of the motion sensor becomes possible by identifying the specific motion pattern the motion sensor is executing at the location where it is attached, e.g. to the athlete or a piece of his sports equipment.

In yet a further embodiment of the configurable motion sensor the motion classification means is located in the sensor hub and is adapted to generate at least part of the configuration data based on an identified motion pattern.

In this way the processing required for motion pattern identification takes place in the sensor hub where electrical power is available more abundantly. Furthermore, once a specific motion pattern has been identified the sensor hub is able to download an appropriate software module from the application server and then upload the necessary signal processing program and/or the associated transmission protocol program to the memory unit.

As a further aspect, a sensor network is provided comprising a plurality of configurable motion sensors and a single, common sensor hub to which all the configurable motion sensors are operationally connected.

In this way the single, common sensor hub can be employed to receive and post-process and/or display and/or store and/or forward to a remote computer or data server the data from the plurality of motion sensors. This opens up the possibility to simultaneously provide various performance measures to the user of the sensor hub or to determine more complex performance measures by the sensor hub based on data from multiple motion sensors measuring different motion parameters. Furthermore, it is possible to simultaneously determine the same performance measure for multiple users by means of a single sensor hub, e.g. for a coach of a cycling team who wants to monitor the pedalling cadence of all the riders in his team centrally from an escort vehicle during a race. Moreover, such a structure also provides redundancy which can be exploited to either derive more accurate performance values or to increase system reliability by being able to substitute faulty sensors with operational back-up sensors on the fly.

As a further aspect, a system is provided for configuring a configurable motion sensor, comprising an application server with a software database, a sensor interfacing device, such as for instance a computer, and the configurable motion sensor, wherein a plurality of software modules, e.g. for sports, fitness, ambulatory monitoring and therapy applications, requiring the motion sensor to determine at least one motion parameter are stored in the software database. Each of the plurality of software modules requiring the motion sensor comprises an application program, a signal processing program and a transmission protocol program. In such a system the sensor interfacing device is operable to download a selected software module via a communication network and to extract the signal processing program and the transmission protocol program from the selected software module and to upload the signal processing program and the transmission protocol program to the configurable motion sensor either via a wireless connection, e.g. based on the ZigBee, Bluetooth, Bluetooth Low Energy (LE), ANT+, Z-Wave, BodyLAN or Toumaz Nano Sensor Protocol (NSP) standard, or a wired connection, e.g. a USB cable.

In this way a wide variety of software modules can be provided in a simple manner for a broad range of motion sensor applications to a versatile configurable motion sensor, which can then be more universally applied than presently available sensor devices which are restricted to a single application and for determining only one motion parameter in a completely predetermined and fixed fashion.

In a further embodiment of the system the sensor interfacing device is operable to send configuration data which determine the signal processing program and/or the transmission protocol program to be used in the configurable motion sensor to the configurable motion sensor.

In this way the sensor interfacing device acts as an intermediary between the application server and the configurable motion sensor and handles the selection, retrieval and distribution of the software module as well as parts thereof.

In yet a further embodiment of the system the sensor interfacing device is further operable to extract the application program from the selected software module and to upload the application program and the transmission protocol program to a sensor hub, such as for instance a sports computer, a mobile phone or a personal digital assistant, intended in be operationally connected with the configurable motion sensor.

In this way the sensor interfacing device also acts as an intermediary between the application server and the sensor hub and further handles the extraction and transfer of parts of the software module to the sensor hub.

In yet a further embodiment of the system the sensor hub is the sensor interfacing device, i.e. performs its tasks, and is operable to execute the application program and the transmission protocol program.

In this way the sensor hub directly downloads the desired software module for a certain application from the application server, extracts the different parts and uploads them to the motion sensor with which it is associated and also executes the application program to post-process/display/store/forward the data, e.g. motion parameters, it receives from the motion sensor.

The invention is defined by claims 1-14.

### BRIEF DESCRIPTION OF THE DRAWINGS

For the purpose of facilitating the understanding of the present invention, exemplary embodiments thereof are illustrated in the accompanying drawings which are to be considered in connection with the following description. In this way the present invention may be more readily understood and appreciated. What is shown in the figures is the following:
- Fig. 1: shows a block diagram of a configurable motion sensor according to the present invention;
- Fig. 2: shows in a schematic representation a system for configuring a configurable motion sensor according to the present invention; and
- Fig. 3: shows in a schematic representation a sensor network with a plurality of configurable motion sensors according to the present invention which are all operationally connected to a single, common sensor hub.

### DETAILED DESCRIPTION OF THE INVENTION

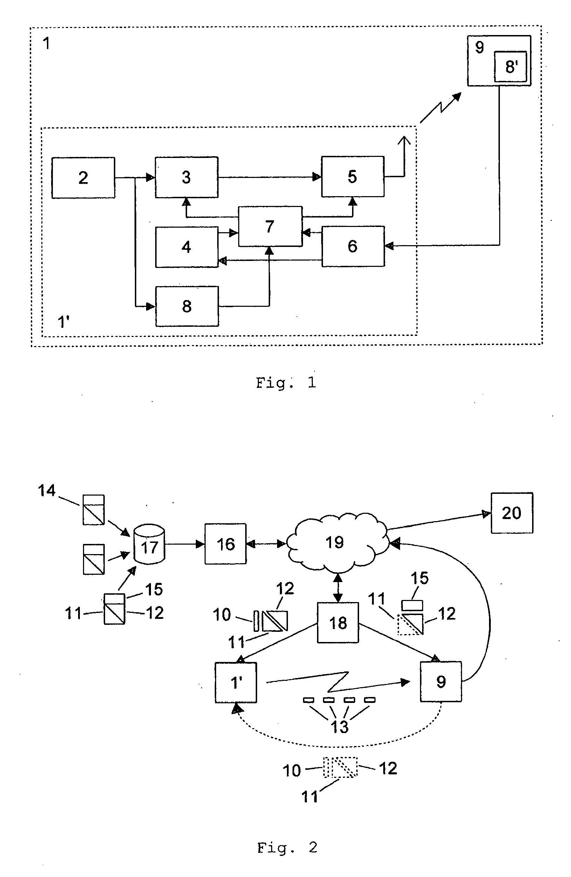

Fig. 1 depicts a block diagram of a configurable motion sensor 1. In the embodiment shown in Fig. 1 the configurable motion sensor 1 includes a sensor device 1' and a separate sensor hub 9, which is remotely located from the sensor device 1' during normal operation of the configurable motion sensor 1, i.e. whilst determining a desired motion parameter. The sensor device 1' comprises an accelerometer 2 and/or a gyroscope 2 as sensor element. Suitable miniature multi-axis accelerometers for motion measurements in a plane or three-dimensional space, respectively, are the dual-axis accelerometer ADXL210 or the 3-axis accelerometer ADXL345, respectively, from Analog Devices, Inc. Suitable miniature gyroscopes for motion sensing are the GYROSTAR piezoelectric vibrating gyroscopes from Murata Manufacturing Co., Ltd.

The one or more output signals from the accelerometer 2 and/or the gyroscope 2 are provided to a processing unit 3 which determines at least one motion parameter using a signal processing program which is stored in the memory unit 4. A plurality of signal processing programs are stored in the memory unit 4 and a program selection means 7 selects the one required for determining the desired motion parameter dependent on configuration data provided to the program selection means 7 from the sensor hub 9 via a receiving unit 6.

The configuration data is transferred from the sensor hub 9 to the sensor device 1' either wirelessly, e.g. based on the ZigBee, Bluetooth, Bluetooth Low Energy (LE), ANT+, Z-Wave, BodyLAN or Toumaz Nano Sensor Protocol (NSP) standard, or through a wired connection, e.g. a USB cable.

The one or more motion parameters determined by the processing unit 3 are subsequently sent to the sensor hub 9 via a wireless transmitting unit 5, which employs a transmission protocol program to do so. The transmission protocol program may be different depending on the type of data to be sent to the sensor hub 9. For instance several different standardised ANT+ "device profiles" exist that define the network parameters and the structure of the data payload for various applications such as stride-based speed and distance monitoring, bicycle speed and cadence monitoring or monitoring a bicycle rider's expended drive power, etc. Such information forms part of the transmission protocol program. Furthermore, the transmission protocol program can also include the type of modulation and coding being employed to send the data. The transmission protocol program to be used to send the data is also determined by the configuration data that is provided to the program selection means 7, which selects the required transmission protocol program from a plurality of transmission protocol programs stored in the memory unit 4. The wireless transmission.scheme can for instance be based on the ZigBee, Bluetooth, Bluetooth Low Energy (LE), ANT+, Z-Wave, BodyLAN or Toumaz Nano Sensor Protocol (NSP) standard.

The sensor hub 9 can request information regarding the signal processing programs and/or the transmission protocol programs presently stored in the memory unit 4. If the sensor hub 9 detects that a program required for a certain desired application is presently not stored in the memory unit 4, it can upload a signal processing program and/or a transmission protocol program to the sensor device 1' via the receiving unit 6. Again, as with the configuration data, this can be done wirelessly or by means of wired link such as a USB cable. The latter is more suitable for uploading programs since it allows a high transmission rate. Once all required programs are stored in the memory unit 4, wireless transmission of the configuration data from the sensor hub 9 to the sensor device 1' is highly suitable since this allows to rapidly re-configure the sensor device 1' without having to physically connect the sensor device 1' to the sensor hub 9.

In a specific embodiment of the configurable motion sensor 1 it further comprises a motion classification means 8, 8'. This motion classification means 8, 8' is capable of identifying a plurality of different motion patterns based on motion features which are extracted from the one or more output signals from the accelerometer 2 and/or the gyroscope 2. The motion classification means 8 can be part of the sensor device 1' in which case the sensor device 1' is capable of "self-configuration" dependent on the identified motion pattern. The motion classification means 8 hence generates at least part of the configuration data and provides this to the program selection means 7, which then selects the signal processing program appropriate to determine at least one motion parameter dependent on the configuration data generated by the motion classification means 8, which in turn is dependent on the motion pattern identified by the motion classification means 8. Alternatively, the motion classification means 8' can be located in the sensor hub 9. This requires sending the output signal(s) from the accelerometer 2 and/or the gyroscope 2 or one or more signals derived therefrom to the sensor hub 9. This off-loads considerable processing from the sensor device 1' to the sensor hub 9 so that a substantial power-savings is achievable at the sensor device 1' where typically less power is available due to space and size limitations for incorporation of e.g. a (rechargeable) battery cell.

Fig. 2 depicts in a schematic representation a system for configuring the configurable motion sensor 1 illustrated in Fig. 1. A user of the configurable motion sensor 1 comprising the sensor device 1' and the associated sensor hub 9 selects a desired application provided by an application server 16 which is connected to a software database 17 containing a wide range of software modules 14 for sports, fitness, ambulatory monitoring and therapy applications. In order to select a specific application the user employs a sensor interfacing device 18 such as a computer, a portable digital assistant (PDA) or a mobile phone. Many services are available meanwhile for downloading software applications (referred to as "apps") over the Internet to mobile devices, e.g. Apple's App Store, Nokia's Ovi Store and the Android Store. The user can select a desired application using the sensor interfacing device 18 from a plurality of applications offered by the application server 16 for instance via a web page of one of the just mentioned online stores. A software module 14 corresponding to the desired application is then downloaded from the software database 17 connected to the application server 16 via a communication network 19 such as the Internet to the sensor interfacing device 18. Each of the software modules 14 requiring the use of a configurable motion sensor 1 comprises multiple components, i.e. a signal processing program 11, a transmission protocol program 12 and an application program 15, which are intended for different parts of the configurable motion sensor 1. The sensor interfacing device 18 is capable of extracting these different components 11, 12 & 15 from the software module 14 and providing them to the parts of the configurable motion sensor 1 for which they are intended. I.e. the sensor interfacing device 18 sends the signal processing program 11 and the transmission protocol program 12 to the sensor device 1'. Moreover, the sensor interfacing device 18 can also send configuration data 10 to the sensor device 1', so that the correct signal processing program 11 is selected for determining the desired motion parameter 13, and that the correct transmission protocol program 12 is selected for sending the determined motion parameter 13 to the sensor hub 9. Furthermore, the sensor interfacing device 18 sends the transmission protocol program 12 and the application program 15 to the sensor hub 9. The sensor hub 9 requires the transmission protocol program 12 in order to be able to receive the motion parameter 13 being sent by the sensor device 1', and requires the application.program 12 in order to be able to post-process and/or display and/or store and/or forward the received motion parameters 13. In the latter case the received motion parameters 13 is forwarded, i.e. uploaded to a remote computer or a data server 20 via the communication network 19, e.g. for centralised storage of the motion parameters or for instance for sharing them amongst a community of users such a group of cyclists or joggers.

Alternatively, instead of utilizing a sensor interfacing device 18, the tasks performed by such a device can be carried out directly by the sensor hub 9. I.e. the sensor hub 9 can download the software module 14 from the application server 16, extract the different components 11, 12 & 15 and subsequently send the signal processing program 11 and/or the transmission protocol program 12 as well as the configuration data 10 to the sensor device 1' as indicated in Fig. 2 by the dashed arrow and the dashed blocks.

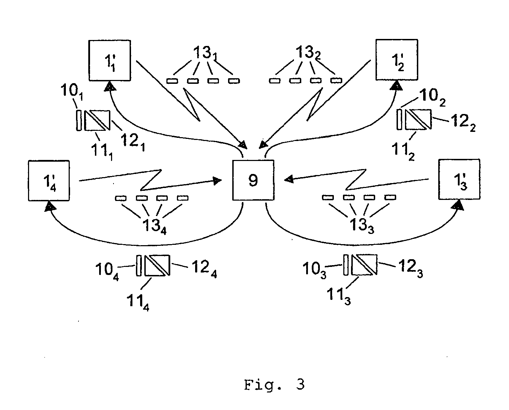

Fig. 3 depicts in.a schematic representation a sensor network comprising multiple sensor devices 1₁', 1₂', 1₃', 1₄' (the subscript representing the sensor device index) which are connected to a single centralised, common sensor hub 9. Different configuration data 10₁', 10₂', 10₃', 10₄' can be sent to each of the plurality of sensor devices 1₁', 1₂', 1₃', 1₄' from the central sensor hub 9 by using an appropriate addressing scheme as part of the transmission protocol. The signal processing programs 11₁', 11₂', 11₃', 11₄' as well as the transmission protocol programs 12₁', 12₂', 12₃', 12₄' can also be distributed to the different sensor devices 1₁', 1₂', 1₃', 1₄' from the central sensor hub 9 by using such an addressing scheme. Each sensor device 1₁', 1₂', 1₃', 1₄' then sends the data that it has determined, i.e. motion parameters 13₁', 13₂', 13₃', 13₄', to the common sensor hub 9, where it is centrally post-processed, displayed to the user of the sensor network, stored and/or uploaded to a remote computer or data server 20 via a communication network 19 (both not shown in Fig. 3; in this regard see Fig. 2).

## Claims

1. A method for configuring a motion sensor (1) comprising an accelerometer (2) and/or a gyroscope (2), a processing unit (3), a memory unit (4), a program selection means (7), a wireless transmitting unit (5), and a receiving unit (6), the method comprising:
- providing configuration data (10) to the program selection means (7);
- selecting by the program selection means (7) a signal processing program (11) from a plurality of signal processing programs (11) stored in the memory unit (4) dependent on the provided configuration data (10);
- determining by the processing unit (3) at least one motion parameter (13) from one or more output signals of the accelerometer (2) and/or the gyroscope (2) using the selected signal processing program (11);
- selecting a transmission protocol program (12) from a plurality of transmission protocol programs (12) stored in the memory unit (4) dependent on the provided configuration data (10); and
- sending by the wireless transmitting unit (5) the at least one motion parameter (13) or the one or more output signals of the accelerometer (2) and/or the gyroscope (2) or one or more signals based on said output signals using the selected transmission protocol program (12),
wherein at least one of the plurality of signal processing programs (11) and/or at least one of the plurality of transmission protocol programs (12) is received by the receiving unit (6).

2. The method of claim 1, wherein the step of providing configuration data (10) comprises:
- receiving by the receiving unit (6) the configuration data (10).

3. The method of claim 1 or 2, wherein the motion sensor (1) further comprises a motion classification means (8), the method further comprising:
- extracting motion features from the one or more output signals of the accelerometer (2) and/or the gyroscope (2) ;
- identifying by the motion classification means (8) a motion pattern from a plurality of motion patterns based on the extracted motion features; and
- generating at least part of the configuration data (10) related to selecting the transmission protocol program (12) from the plurality of transmission protocol programs (12) stored in the memory unit (4) to be provided to the program selection means (7) dependent on the identified motion pattern.

4. The method of claim 1, further comprising:
- downloading a software module (14), which comprises at least a signal processing program (11) and a transmission protocol program (12), from an application server (16) to a sensor interfacing device (18) or sensor hub (9), such as for instance a sports computer, a mobile phone or a portable digital assistant;
- extracting the signal processing program (11) and/or the transmission protocol program (12) from the software module (14); and
- sending the signal processing program (11) and/or the transmission protocol program (12) from the sensor interfacing device (18) or sensor hub (9) to the receiving unit (6).

5. The method of claim 4, further comprising receiving by the sensor hub (9) using the transmission protocol program (12) the at least one motion parameter (13) or the one or more output signals of the accelerometer (2) and/or the gyroscope (2) or one or more signals based on said output signals from the wireless transmitting unit (5), and wherein the software module (14) further comprises an application program (15), and wherein the method further comprises extracting the application program (15) from the software module (14) and executing by the sensor hub (9) the application program (15) for post-processing and/or displaying and/or storing the received at least one motion parameter (13) or the one or more output signals of the accelerometer (2) and/or the gyroscope (2) or one or more signals based on said output signals from the wireless transmitting unit (5).

6. The method of claim 4 or 5, further comprising sending by the wireless transmitting unit (5) information regarding one or more of the following to the sensor hub (9):
- configuration data (10);
- identification number, such as a serial number or address data, of the motion sensor (1);
- list of signal processing programs (11) stored in the memory unit (4);
- list of transmission protocol programs (12) stored in the memory unit (4);
- information regarding one or more of the signal processing programs (11) or one or more of the transmission protocol programs (12) stored in the memory unit (4), for instance a checksum or a version number;
- information regarding the selected signal processing program (11) or the selected transmission protocol program (12).

7. A configurable motion sensor (1) comprising an accelerometer (2) and/or a gyroscope (2), a processing unit (3), a memory unit (4), a wireless transmitting unit (5), a receiving unit (6) and a program selection means (7), wherein the processing unit (3) is connected to the accelerometer (2) and/or the gyroscope (2), the memory unit (4), the wireless transmitting unit (5), the receiving unit (6) and the program selection means (7), and wherein the receiving unit (6) is operable to receive configuration data (10), and wherein the processing unit (3) is operable to determine at least one motion parameter (10) from one or more output signals of the accelerometer (2) and/or the gyroscope (2) using one of a plurality of signal processing programs (11) storable in the memory unit (4) and selectable by the program selection means (7) dependent on the configuration data (10), **characterised in that** the wireless transmitting unit (5) is operable to transmit the at least one motion parameter (10) using one of a plurality of transmission protocol programs (12) storable in the memory unit (4) and selectable by the program selection means (7) dependent on the configuration data (10), and wherein the receiving unit (6) is operable to receive at least one of the plurality of signal processing programs (11) and/or at least one of the plurality of transmission protocol programs (12).

8. The configurable motion sensor (1) of claim 7, further comprising a separate sensor hub (9), such as for instance a sports computer, a mobile phone or a personal digital assistant, wherein the wireless transmitting unit (5) is wirelessly connected to the sensor hub (9), and wherein the sensor hub (9) is adapted to receive one or more of the following from the wireless transmitting unit (5):
- configuration data (10);
- identification number, such as a serial number or address data, of the motion sensor (1);
- list of signal processing programs (11) stored in the memory unit (4);
- list of transmission protocol programs (12) stored in the memory unit (4);
- information regarding one or more of the signal processing programs (11) or one or more of the transmission protocol programs (12) stored in the memory unit (4), for instance a checksum or a version number;
- information regarding the selected signal processing program (11) or the selected transmission protocol program (12).

9. The configurable motion sensor (1) of claim 7 or 8, wherein the sensor hub (9) is connected to the receiving unit (6) via a wireless connection or via a wired connection and wherein the sensor hub (9) is further adapted to perform one or more of the following:
- send configuration data (10);
- send a signal processing program (11);
- send a transmission protocol program (12);
to the receiving unit (6).

10. The configurable motion sensor (1) of one of the claims 7 to 9, further comprising motion classification means (8) capable of identifying a plurality of motion patterns based on motion features extractable from the one or more output signals of the accelerometer (2) and/or the gyroscope (2), wherein the motion classification means (8) is connected to the program selection means (7) and determines the transmission protocol program (12) to be employed for transmitting the at least one motion parameter (10).

11. A system operable to configure a configurable motion sensor (1') according to one of the claims 7 to 10, the system comprising an application server (16) with a software database (17), a sensor interfacing device (18), such as for instance a computer, and the configurable motion sensor (1'), wherein a plurality of software modules (14) requiring the configurable motion sensor (1') to determine at least one motion parameter (10) are stored in the software database (17), and wherein each of the plurality of software modules (14) requiring the configurable motion sensor (1') comprises an application program (15), a signal processing program (11) and a transmission protocol program (12), and wherein the sensor interfacing device (18) is operable to download a selected software module (14) via a communication network (19) and to extract the signal processing program (11) and the transmission protocol program (12) from the selected software module (14) and to upload the signal processing program (11) and the transmission protocol program (12) to the configurable motion sensor (1').

12. The system of claim 11, wherein the sensor interfacing device (18) is operable to send configuration data (10) which determine the signal processing program (11) and/or the transmission protocol program (12) to be used in the configurable motion sensor (1') to the configurable motion sensor (1').

13. The system of claim 11 or 12, wherein the sensor interfacing device (18) is further operable to extract the application program (15) from the selected software module (14) and to upload the application program (15) and the transmission protocol program (12) to a sensor hub (9), such as for instance a sports computer, a mobile phone or a personal digital assistant, intended in be operationally connected with the configurable motion sensor (1').

14. The system of one of the claims 11 to 13, wherein the sensor hub (9) is the sensor interfacing device (18), and wherein the sensor hub (9) is operable to execute the application program (15) and the transmission protocol program (12).

## Patentansprüche

1. Ein Verfahren zum Konfigurieren eines Bewegungssensors (1) umfassend ein Beschleunigungsmesser (2) und/oder ein Kreisel (2), eine Verarbeitungseinheit (3), eine Speichereinheit (4), ein Programmauswahlmittel (7), eine drahtlose Sendeeinheit (5) und eine Empfängereinheit (6), wobei das Verfahren folgende Schritte umfasst:
- Bereitstellen von Konfigurationsdaten (10) für das Programmauswahlmittel (7);
- Auswählen mittels des Programmauswahlmittels (7) eines Signalverarbeitungsprogramms (11) aus einer Vielzahl von Signalverarbeitungsprogrammen (11), welche in der Speichereinheit (4) gespeichert sind, in Abhängigkeit der bereitgestellten Konfigurationsdaten (10);
- Bestimmen mittels der Verarbeitungseinheit (3) von mindestens einem Bewegungsparameter (13) aus einem oder mehreren Ausgangssignalen des Beschleunigungsmessers (2) und/oder des Kreisels (2) mittels des ausgewählten Signalverarbeitungsprogramms (11);
- Auswählen eines Übertragungsprotokollprogramms (12) aus einer Vielzahl von Übertragungsprotokollprogrammen (12), welche in der Speichereinheit (4) gespeichert sind, in Abhängigkeit der bereitgestellten Konfigurationsdaten (10);
- Senden mittels der drahtlosen Sendeeinheit (5) des mindestens einen Bewegungsparameters (13) oder des einen oder der mehreren Ausgangssignale des Beschleunigungsmessers (2) und/oder des Kreisels (2) oder eines oder mehrere Signale, welche auf den besagten Ausgangssignalen basieren, mittels des ausgewählten Übertragungsprotokollprogramms (12),
wobei mindestens eines aus der Vielzahl von Signalverarbeitungsprogrammen (11) und/oder mindestens eines aus der Vielzahl von Übertragungsprotokollprogrammen (12) mittels der Empfängereinheit (6) empfangen wird.

2. Das Verfahren nach Anspruch 1, wobei der Schritt des Bereitstellens von Konfigurationsdaten (10) folgendes umfasst:
- Empfangen mittels der Empfängereinheit (6) der Konfigurationsdaten (10).

3. Das Verfahren nach Anspruch 1 oder 2, wobei der Bewegungssensor (1) weiter ein Bewegungsklassifikationsmittel (8) umfasst, und wobei das Verfahren weiter folgende Schritte umfasst:
- Extrahieren eines Bewegungsmerkmals aus einem oder mehreren Ausgangssignalen des Beschleunigungsmessers (2) und/oder des Kreisels (2);
- Identifizieren mittels des Bewegungsklassifikationsmittels (8) eines Bewegungsmusters aus einer Vielzahl von Bewegungsmustern basierend auf den extrahierten Bewegungsmerkmalen; und
- Generieren von mindestens einem Teil der Konfigurationsdaten (10) betreffend das Auswählen des Übertragungsprotokollprogramms (12) aus der Vielzahl von Übertragungsprotokollprogrammen (12), welche in der Speichereinheit (4) gespeichert sind, welcher dem Programmauswahlmittel (7) in Abhängigkeit des identifizierten Bewegungsmusters bereitgestellt wird.

4. Das Verfahren nach Anspruch 1, weiter umfassend folgende Schritte:
- Herunterladen eines Softwaremoduls (14), welches mindestens ein Signalverarbeitungsprogramm und ein Übertragungsprotokollprogramm (12) umfasst, von einem Applikationsserver (16) zu einem Sensorschnittstellengerät (18) oder einem Sensorknoten (9), wie beispielsweise ein Sportcomputer, ein Mobiltelefon oder ein portabler digitaler Assistent;
- Extrahieren des Signalverarbeitungsprogramms (11) und/oder des Übertragungsprotokollprogramms (12) aus dem Softwaremodul (14); und
- Senden des Signalverarbeitungsprogramms (11) und/oder des Übertragungsprotokollprogramms (12) von dem Sensorschnittstellengerät (18) oder dem Sensorknoten (9) an die Empfängereinheit (6).

5. Das Verfahren nach Anspruch 4, weiter umfassend Empfangen mittels des Sensorknotens (9) mittels des Übertragungsprotokollprogramms (12) des mindestens einen Bewegungsparameters (13) oder des einen oder der mehreren Ausgangssignale des Beschleunigungsmessers (2) und/oder des Kreisels (2) oder eines oder mehrere Signale, welche auf den besagten Ausgangssignalen basieren, von der drahtlosen Sendeeinheit (5), und wobei das Softwaremodul (14) weiter ein Applikationsprogramm (15) umfasst, und wobei das Verfahren weiter umfasst Extrahieren des Applikationsprogramms (15) aus dem Softwaremodul (14) und Ausführen des Applikationsprogramms (15) mittels des Sensorknotens (9) zur Nachverarbeitung und/oder zur Anzeige und/oder zum Speichern des empfangenen mindestens einen Bewegungsparameters (13) oder des einen oder der mehreren Ausgangssignale des Beschleunigungsmessers (2) und/oder des Kreisels (2) oder eines oder mehrere Signale, welche auf den besagten Ausgangssignalen basieren, von der drahtlosen Sendeeinheit (5).

6. Das Verfahren nach Anspruch 4 oder 5, weiter umfassend Senden mittels der drahtlosen Sendeeinheit (5) von Informationen betreffend eines oder mehrerer der folgenden zum Sensorknoten (9):
- Konfigurationsdaten (10);
- Identifikationsnummer, wie eine Seriennummer oder Adressdaten, des Bewegungssensors (1);
- Liste von Signalverarbeitungsprogrammen (11), welche in der Speichereinheit (4) gespeichert sind;
- Liste von Übertragungsprotokollprogrammen (11), welche in der Speichereinheit (4) gespeichert sind;
- Informationen betreffend eines oder mehrere der Signalverarbeitungsprogramme (11) oder eines oder mehrere der Übertragungsprotokollprogramme (12), welche in der Speichereinheit (4) gespeichert sind, beispielsweise eine Checksumme oder eine Versionennummer;
- Informationen betreffend das ausgewählte Signalverarbeitungsprogramm (11) oder das ausgewählte Übertragungsprotokollprogramm (12).

7. Ein konfigurierbarer Bewegungssensor (1) umfassend ein Beschleunigungsmesser (2) und/oder ein Kreisel (2), eine Verarbeitungseinheit (3), eine Speichereinheit (4), ein Programmauswahlmittel (7), eine drahtlose Sendeeinheit (5) und eine Empfängereinheit (6), wobei die Verarbeitungseinheit (3) mit dem Beschleunigungsmesser (2) und/oder dem Kreisel (2), der Speichereinheit (4), der drahtlose Sendeeinheit (5), der Empfängereinheit (6) und dem Programmauswahlmittel (7) verbunden ist, und wobei die Empfängereinheit (6) betreibbar ist um Konfigurationsdaten (10) zu empfangen, und wobei die Verarbeitungseinheit (3) betreibbar ist um mindestens einen Bewegungsparameter (13) aus einem oder mehreren Ausgangssignalen des Beschleunigungsmessers (2) und/oder des Kreisels (2) mittels eines einer Vielzahl von Signalverarbeitungsprogrammen (11) zu bestimmen, welche in der Speichereinheit (4) speicherbar und mittels des Programmauswahlmittels (7) in Abhängigkeit der Konfigurationsdaten (10) auswählbar sind, **dadurch gekennzeichnet, dass** die drahtlose Sendeeinheit (5) betreibbar ist um den mindestens einen Bewegungsparameter (10) mittels eines einer Vielzahl von Übertragungsprotokollprogrammen (12) zu senden, welche in der Speichereinheit (4) speicherbar und mittels des Programmauswahlmittels (7) in Abhängigkeit der Konfigurationsdaten (10) auswählbar sind, und wobei die Empfängereinheit (6) betreibbar ist um mindestens eines aus der Vielzahl von Signalverarbeitungsprogrammen (11) und/oder eines aus der Vielzahl von Übertragungsprotokollprogrammen (12) zu empfangen.

8. Der konfigurierbare Bewegungssensor (1) nach Anspruch 7, weiter umfassend einen separaten Sensorknoten (9), wie beispielsweise ein Sportcomputer, ein Mobiltelefon oder ein portabler digitaler Assistent, wobei die drahtlose Sendeeinheit (5) drahtlos mit dem Sensorknoten (9) verbunden ist, und wobei der Sensorknoten (9) ausgebildet ist um eines oder mehrere der folgenden von der drahtlosen Sendeeinheit (5) zu empfangen:
- Konfigurationsdaten (10);
- Identifikationsnummer, wie eine Seriennummer oder Adressdaten, des Bewegungssensors (1);
- Liste von Signalverarbeitungsprogrammen (11), welche in der Speichereinheit (4) gespeichert sind;
- Liste von Übertragungsprotokollprogrammen (12), welche in der Speichereinheit (4) gespeichert sind;
- Informationen betreffend eines oder mehrere der Signalverarbeitungsprogrammen (11) oder eines oder mehrere der Übertragungsprotokollprogrammen (12), welche in der Speichereinheit (4) gespeichert sind, beispielsweise eine Checksumme oder eine Versionennummer;
- Informationen betreffend das ausgewählte Signalverarbeitungsprogramm (11) oder das ausgewählte Übertragungsprotokollprogramm (12).

9. Der konfigurierbare Bewegungssensor (1) nach Anspruch 7 oder 8, wobei der Sensorknoten (9) mit der Empfängereinheit (6) via eine drahtlose oder eine drahtgebundene Verbindung verbunden ist, und wobei der Sensorknoten (9) weiter ausgebildet ist um eines oder mehrere der folgenden auszuführen:
- Senden von Konfigurationsdaten (10);
- Senden eines Signalverarbeitungsprogramms (11);
- Senden eines Übertragungsprotokollprogramms (12);
an die Empfängereinheit (6).

10. Der konfigurierbare Bewegungssensor (1) nach einem der Ansprüche 7 bis 9, weiter umfassend ein Bewegungsklassifikationsmittel (8), welches imstande ist, eine Vielzahl von Bewegungsmustern zu identifizieren basierend auf Bewegungsmerkmalen, welche extrahierbar sind aus einem oder mehreren Ausgangssignalen des Beschleunigungsmessers (2) und/oder des Kreisels (2), wobei das Bewegungsklassifikationsmittel (8) mit dem Programmauswahlmittel (7) verbunden ist und das Übertragungsprotokollprogramm (12) bestimmt, welches zum Senden des mindestens einen Bewegungsparameters (10) verwendet wird.

11. Ein System, welches betreibbar ist um einen konfigurierbaren Bewegungssensor (1') nach einem der Ansprüche 7 bis 10 zu konfigurieren, wobei das System ein Applikationsserver (16) mit einer Softwaredatenbank (17), ein Sensorschnittstellengerät (18), wie beispielsweise ein Computer, und den konfigurierbaren Bewegungssensor (1') umfasst, wobei eine Vielzahl von Softwaremodulen (14), welche den konfigurierbaren Bewegungssensor (1') benötigen um mindestens einen Bewegungsparameter (10) zu bestimmen, in der Softwaredatenbank (17) gespeichert sind, und wobei jedes der Vielzahl von Softwaremodulen (14), welche den konfigurierbaren Bewegungssensor (1') benötigen, ein Applikationsprogramm (15), ein Signalverarbeitungsprogramm (11) und ein Übertragungsprotokollprogramm (12) umfasst, und wobei das Sensorschnittstellengerät (18) betreibbar ist um ein ausgewähltes Softwaremodul (14) via ein Kommunikationsnetzwerk (19) herunterzuladen, und das Signalverarbeitungsprogramm (11) und das Übertragungsprotokollprogramm (12) aus dem ausgewählten Softwaremodul (14) zu extrahieren und das Signalverarbeitungsprogramm (11) und das Übertragungsprotokollprogramm (12) auf den konfigurierbaren Bewegungssensor (1') hochzuladen.

12. Das System nach Anspruch 11, wobei das Sensorschnittstellengerät (18) betreibbar ist um Konfigurationsdaten (10), welche bestimmen, welches Signalverarbeitungsprogramm (11) und/oder welches Übertragungsprotokollprogrammen (12) im konfigurierbaren Bewegungssensor (1') verwendet wird, an den konfigurierbaren Bewegungssensor (1') zu senden.

13. Das System nach Anspruch 11 oder 12, wobei das Sensorschnittstellengerät (18) weiter betreibbar ist um das Applikationsprogramm (15) aus dem ausgewählten Softwaremodul (14) zu extrahieren und das Applikationsprogramm (15) und das Übertragungsprotokollprogramm (12) auf einen Sensorknoten (9), wie beispielsweise ein Sportcomputer, ein Mobiltelefon oder ein portabler digitaler Assistent, welcher vorgesehen ist um mit dem konfigurierbaren Bewegungssensor (1') wirkverbunden zu sein, hochzuladen.

14. Das System nach einem der Ansprüche 11 bis 13, wobei der Sensorknoten (9) das Sensorschnittstellengerät (18) ist, und wobei der Sensorknoten (9) betreibbar ist um das Applikationsprogramm (15) und das Übertragungsprotokollprogramm (12) auszuführen.

## Revendications

1. Procédé de configuration d'un capteur de mouvement (1) comprenant un accéléromètre (2) et/ou un gyroscope (2), une unité de traitement (3), une unité de mémoire (4), un moyen de sélection de programme (7), une unité de transmission sans fil (5) et une unité de réception (6), le procédé consistant à:
- fournir des données de configuration (10) au moyen de sélection de programme (7);
- sélectionner, à l'aide du moyen de sélection de programme (7), un programme de traitement de signal (11) parmi une pluralité de programmes de traitement de signal (11) stockés dans l'unité de mémoire (4) en fonction des données de configuration fournies (10);
- déterminer, à l'aide de l'unité de traitement (3), au moins un paramètre de mouvement (13) à partir d'un ou de plusieurs signaux de sortie de l'accéléromètre (2) et/ou du gyroscope (2) à l'aide du programme de traitement de signal sélectionné (11);
- sélectionner un programme de protocole de transmission (12) parmi une pluralité de programmes de protocole de transmission (12) stockés dans l'unité de mémoire (4) en fonction des données de configuration fournies (10); et
- envoyer par le biais de l'unité de transmission sans fil (5) le au moins un paramètre de mouvement (13) ou le ou les signaux de sortie de l'accéléromètre (2) et/ou du gyroscope (2) ou un ou plusieurs signaux sur la base desdits signaux de sortie à l'aide du programme de protocole de transmission sélectionné (12),
dans lequel au moins un programme parmi la pluralité de programmes de traitement de signal (11) et/ou au moins un programme parmi la pluralité de programmes de protocole de transmission (12) est reçu par l'unité de réception (6).

2. Procédé selon la revendication 1, dans lequel l'étape visant à fournir des données de configuration (10) consiste à:
- recevoir par l'unité de réception (6) les données de configuration (10).

3. Procédé selon la revendication 1 ou 2, dans lequel le capteur de mouvement (1) comprend en outre un moyen de classification de mouvement (8), le procédé consistant en outre à:
- extraire des caractéristiques de mouvement à partir du ou des signaux de sortie de l'accéléromètre (2) et/ou du gyroscope (2);
- identifier par le biais du moyen de classification de mouvement (8) un modèle de mouvement parmi une pluralité de modèles de mouvement sur la base des caractéristiques de mouvement extraites; et
- générer au moins une partie des données de configuration (10) correspondantes en sélectionnant le programme de protocole de transmission (12) parmi la pluralité de programmes de protocole de transmission (12) stockés dans l'unité de mémoire (4) à fournir au moyen de sélection de programme (7) en fonction du modèle de mouvement identifié.

4. Procédé selon la revendication 1, consistant en outre à:
- télécharger un module logiciel (14) qui comprend au moins un programme de traitement de signal (11) et un programme de protocole de transmission (12), à partir d'un serveur d'application (16) vers un dispositif d'interface de capteur (18) ou vers un concentrateur de capteurs (9), comme par exemple un ordinateur de sport, un téléphone mobile ou un assistant numérique portable;
- extraire le programme de traitement de signal (11) et/ou le programme de protocole de transmission (12) du module logiciel (14); et
- envoyer le programme de traitement de signal (11) et/ou le programme de protocole de transmission (12) depuis le dispositif d'interface de capteur (18) ou le concentrateur de capteurs (9) vers l'unité de réception (6).

5. Procédé selon la revendication 4, consistant en outre à recevoir par le concentrateur de capteurs (9) à l'aide du programme de protocole de transmission (12), le au moins un paramètre de mouvement (13) ou le ou les signaux de sortie de l'accéléromètre (2) et/ou du gyroscope (2) ou un ou plusieurs signaux basés sur lesdits signaux de sortie de l'unité de transmission sans fil (5), et dans lequel le module logiciel (14) comprend en outre un programme d'application (15), et dans lequel le procédé consiste en outre à extraire le programme d'application (15) du module logiciel (14) et à exécuter par le concentrateur de capteurs (9) le programme d'application (15) pour le post-traitement et/ou pour afficher et/ou pour stocker le au moins un paramètre de mouvement (13) reçu ou le ou les signaux de sortie de l'accéléromètre (2) et/ou du gyroscope (2) ou un ou plusieurs signaux basés sur lesdits signaux de sortie à partir de l'unité de transmission sans fil (5).

6. Procédé selon la revendication 4 ou 5, consistant en outre à envoyer par le biais de l'unité de transmission sans fil (5) des informations concernant un ou plusieurs des éléments suivants vers le concentrateur de capteurs (9) :
- des données de configuration (10);
- un numéro d'identification, comme un numéro de série ou des données d'adresse, du capteur de mouvement (1);
- une liste de programmes de traitement de signal (11) stockés dans l'unité de mémoire (4);
- une liste de programmes de protocole de transmission (12) stockés dans l'unité de mémoire (4);
- des informations concernant un ou plusieurs des programmes de traitement de signal (11) ou un ou plusieurs des programmes de protocole de transmission (12) stockés dans l'unité de mémoire (4), par exemple une somme de contrôle ou un numéro de version;
- des informations concernant le programme de traitement de signal sélectionné (11) ou le programme de protocole de transmission sélectionné (12).

7. Capteur de mouvement configurable (1) comprenant un accéléromètre (2) et/ou un gyroscope (2), une unité de traitement (3), une unité de mémoire (4), une unité de transmission sans fil (5), une unité de réception (6) et un moyen de sélection de programme (7), dans lequel l'unité de traitement (3) est raccordée à l'accéléromètre (2) et/ou au gyroscope (2), à l'unité de mémoire (4), à l'unité de transmission sans fil (5), à l'unité de réception (6) et au moyen de sélection de programme (7), et dans lequel l'unité de réception (6) peut être utilisée pour recevoir des données de configuration (10) et dans lequel l'unité de traitement (3) peut être utilisée pour déterminer au moins un paramètre de mouvement (10) à partir d'un ou de plusieurs signaux de sortie de l'accéléromètre (2) et/ou du gyroscope (2) à l'aide d'un programme parmi une pluralité de programmes de traitement de signal (11) pouvant être stockés dans l'unité de mémoire (4) et pouvant être sélectionnés par le moyen de sélection de programme (7) en fonction des données de configuration (10), **caractérisé en ce que** l'unité de transmission sans fil (5) peut être utilisée pour transmettre le au moins un paramètre de mouvement (10) à l'aide d'un programme parmi une pluralité de programmes de protocole de transmission (12) pouvant être stockés dans l'unité de mémoire (4) et pouvant être sélectionnés par le moyen de sélection de programme (7) en fonction des données de configuration (10), et dans lequel l'unité de réception (6) peut être utilisée pour recevoir au moins un programme parmi la pluralité de programmes de traitement de signal (11) et/ou au moins un programme parmi la pluralité de programmes de protocole de transmission (12).

8. Capteur de mouvement configurable (1) selon la revendication 7, comprenant en outre un concentrateur de capteurs (9) séparé, comme par exemple un ordinateur de sport, un téléphone mobile ou un assistant personnel numérique, dans lequel l'unité de transmission sans fil (5) est raccordée sans fil au concentrateur de capteurs (9), et dans lequel le concentrateur de capteurs (9) est adapté pour recevoir un ou plusieurs des éléments suivants depuis l'unité de transmission sans fil (5):
- des données de configuration (10);
- un numéro d'identification, comme un numéro de série ou des données d'adresse, du capteur de mouvement (1);
- une liste de programmes de traitement de signal (11) stockés dans l'unité de mémoire (4);
- une liste de programmes de protocole de transmission (12) stockés dans l'unité de mémoire (4);
- des informations concernant un ou plusieurs des programmes de traitement de signal (11) ou un ou plusieurs des programmes de protocole de transmission (12) stockés dans l'unité de mémoire (4), par exemple une somme de contrôle ou un numéro de version;
- des informations concernant le programme de traitement de signal sélectionné (11) ou le programme de protocole de transmission sélectionné (12).

9. Capteur de mouvement configurable (1) selon la revendication 7 ou 8, dans lequel le concentrateur de capteurs (9) est raccordé à l'unité de réception (6) via une connexion sans fil ou via une connexion filaire et dans lequel le concentrateur de capteurs (9) est en outre adapté pour réaliser une ou plusieurs des actions suivantes:
- envoyer des données de configuration (10);
- envoyer un programme de traitement de signal (11);
- envoyer un programme de protocole de transmission (12);
à l'unité de réception (6).

10. Capteur de mouvement configurable (1) selon l'une des revendications 7 à 9, comprenant en outre un moyen de classification de mouvement (8) capable d'identifier une pluralité de modèles de mouvement sur la base de caractéristiques de mouvement pouvant être extraites depuis le ou les signaux de sortie de l'accéléromètre (2) et/ou du gyroscope (2), dans lequel le moyen de classification de mouvement (8) est raccordé au moyen de sélection de programme (7) et détermine le programme de protocole de transmission (12) à utiliser pour transmettre le au moins un paramètre de mouvement (10).

11. Système pouvant être utilisé pour configurer un capteur de mouvement configurable (1') selon l'une des revendications 7 à 10, le système comprenant un serveur d'application (16) avec une base de données logicielle (17), un dispositif d'interface de capteur (18), comme par exemple un ordinateur, et le capteur de mouvement configurable (1'), dans lequel une pluralité de modules logiciels (14) nécessitant le capteur de mouvement configurable (1') pour déterminer au moins un paramètre de mouvement (10) sont stockés dans la base de données logicielle (17), et dans lequel chacune des pluralités de modules logiciels (14) nécessitant le capteur de mouvement configurable (1') comprend un programme d'application (15), un programme de traitement de signal (11) et un programme de protocole de transmission (12), et dans lequel le dispositif d'interface de capteur (18) peut être utilisé pour télécharger un module logiciel sélectionné (14) via un réseau de communication (19) et pour extraire le programme de traitement de signal (11) et le programme de protocole de transmission (12) depuis le module logiciel sélectionné (14) et de télécharger le programme de traitement de signal (11) et le programme de protocole de transmission (12) vers le capteur de mouvement configurable (1').

12. Système selon la revendication 11, dans lequel le dispositif d'interface de capteur (18) peut être utilisé pour envoyer au capteur de mouvement configurable (1') des données de configuration (10) qui déterminent le programme de traitement de signal (11) et/ou le programme de protocole de transmission (12) à utiliser dans le capteur de mouvement configurable (1').

13. Système selon la revendication 11 ou 12, dans lequel le dispositif d'interface de capteur (18) peut en outre être utilisé pour extraire le programme d'application (15) du module logiciel sélectionné (14) et pour télécharger le programme d'application (15) et le programme de protocole de transmission (12) vers un concentrateur de capteurs (9), comme par exemple un ordinateur de sport, un téléphone mobile ou un assistant numérique personnel, destiné à être raccordé de façon opérationnelle au capteur de mouvement configurable (1').

14. Système selon l'une des revendications 11 à 13, dans lequel le concentrateur de capteurs (9) est le dispositif d'interface de capteur (18) et dans lequel le concentrateur de capteurs (9) peut être utilisé pour exécuter le programme d'application (15) et le programme de protocole de transmission (12).
